# EUROPÄISCHE PATENTANMELDUNG

(11) **EP 1 213 286 A1**
(43) Veröffentlichungstag der Anmeldung: **12.06.2002**
(21) Anmeldenummer: 01127421.4
(22) Anmeldetag: 26.11.2001
(51) Int. Cl.: C07D 277/32

(54) **Bis-(2-chlor-thiazolyl-5-methyl)amin sowie Verfahren zur Aufarbeitung von 5-Aminomethyl-2-chlor-thiazol**

(30) Priorität: 08.12.2000 DE 10061083
(71) Anmelder: Bayer Aktiengesellschaft, 51368 Leverkusen (DE)
(72) Erfinder: Rauchschwalbe, Günter, Dr., 51375 Leverkusen (DE)

(57) **Zusammenfassung**

Durch eine spezielle Fällung gelingt es 5-Aminomethyl-2-chlor-thiazol und Bis-(2-chlor-thiazolyl-5-methyl)-amin enthaltende Reaktionsgemische vorteilhaft aufzuarbeiten und Bis-(2-chlor-thiazolyl-5-methyl)-amin oder seine Salze zu isolieren.

## Beschreibung

Die Erfindung betrifft ein Verfahren zur Aufarbeitung von 5-Aminomethyl-2-chlorthiazol und Bis-(2-chlor-thiazolyl-5-methyl)-amin enthaltenden Reaktionsgemischen sowie Bis-(2-chlor-thiazolyl-5-methyl)-amin und seine Salze.

5-Aminomethyl-2-chlor-thiazol (im folgenden auch AMCT genannt) ist ein Vorprodukt zur Herstellung von Insektiziden und daher ein technisch bedeutsames Zwischenprodukt. Aus EP-A-0 446 913 und DE-A-196 53 586 ist es bekannt, dass AMCT durch Umsetzung von 5-Chlormethyl-2-chlorthiazol (im folgenden auch CCMT genannt) mit Ammoniak gemäß dem nachfolgenden Reaktionsschema (I) hergestellt werden kann.

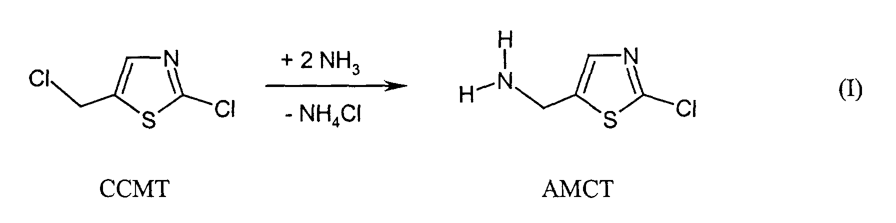

Üblicherweise wird das AMCT aus dem bei der Umsetzung entstehenden Reaktionsgemisch durch Destillation oder Extraktion abgetrennt. Bewährt hat sich hierbei besonders die mehrfache pH-kontrollierte Extraktion mit Dichlormethan (siehe Beispiele der DE-A-196 535 86). Die Verwendung von Methylenchlorid ist im technischen Maßstab jedoch unerwünscht, da es leicht flüchtig und möglicherweise krebserzeugend ist (Kategorie K3). Außerdem ist eine bis zu sechsfache Extraktion im technischen Maßstab mit erheblichem Aufwand verbunden; aus Kosten- und Umweltschutzgründen muss das Lösungsmittel zurückgewonnen und gereinigt werden, was wiederum mit Aufwand verbunden ist.

Gegebenenfalls muss das AMCT-Rohprodukt, wie in EP-A-0 446 913 beschrieben, sogar noch zusätzlich durch präparative Chromatografie gereinigt werden. Die Notwendigkeit einer präparativen Chromatografie mindert die wirtschaftliche Attraktivität des Gesamtverfahrens jedoch erheblich.

Hinzu kommt, dass die bei den Aufarbeitungsverfahren des Standes der Technik anfallenden Rückstände bisher keiner stofflichen Weiterverarbeitung zugeführt wurden.

Die Aufgabe der vorliegenden Erfindung bestand somit darin, ein verbessertes Verfahren zur Aufarbeitung von 5-Aminomethyl-2-chlor-thiazol enthaltenden Reaktionsgemischen zur Verfügung zu stellen, die durch Umsetzung von 5-Chlormethyl-2-chlorthiazol mit Ammoniak erhalten werden können.

Überraschenderweise wurde nun gefunden, dass sich bei der Umsetzung von 5-Chlormethyl-2-chlorthiazol mit Ammoniak außer dem gewünschten Produkt AMCT als Hauptnebenprodukt Bis-(2-chlor-thiazolyl-5-methyl)-amin (Bis-CTMA) der allgemeinen Formel (II) bildet 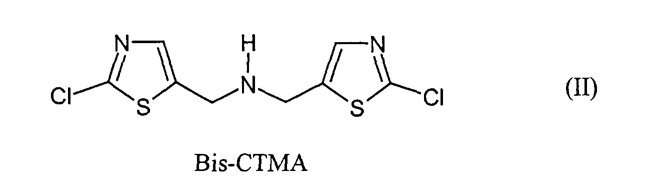 und dass eine wesentlich verbesserte Aufarbeitung derartiger Reaktionsgemische durch Abtrennung dieses Bis-CTMA in Form seiner Salze möglich wird.

Gegenstand der Erfindung ist ein Verfahren zur Aufarbeitung von 5-Aminomethyl-2-chlor-thiazol und Bis-(2-chlor-thiazolyl-5-methyl)-amin enthaltenden Reaktionsgemischen, die durch Umsetzung von 5-Chlormethyl-2-chlorthiazol mit Ammoniak erhältlich sind, dadurch gekennzeichnet, dass
1) gegebenenfalls im Reaktionsgemisch vorhandener Ammoniak abgetrennt wird,
2) das Reaktionsgemisch mit einer derartigen Menge an Wasser und einer derartigen Menge einer anorganischen Säure HX versetzt wird, dass mindestens 85 % der Gesamtmenge des gebildeten Salzes des Bis-(2-chlorthiazolyl-5-methyl)-amins ausfällt und mindestens 85 % der Gesamtmenge des gebildeten Salzes des 5-Aminomethyl-2-chlor-thiazols in Lösung verbleiben, und
3) das ausgefällte Salz des Bis-(2-chlor-thiazolyl-5-methyl)-amins abgetrennt wird.

Gegenstand der Erfindung ist ferner Bis-(2-chlor-thiazolyl-5-methyl)amin der allgemeinen Formel (II) 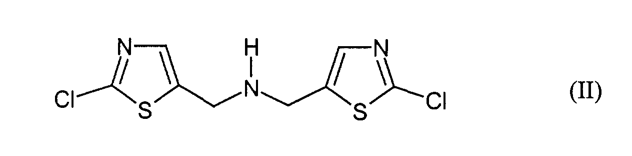 und seine Salze.

Bevorzugt ist hierbei vor allem das Chlorid und das Sulfat des Bis-CTMA.

Sofern das im erfindungsgemäßen Verfahren einzusetzende Reaktionsgemisch aus der vorhergehenden Umsetzung noch überschüssigen Ammoniak enthält, wird dieser zunächst aus dem Reaktionsgemisch abgetrennt. Dies erfolgt üblicherweise durch Destillation. Auf dem gleichen Weg kann auch eventuell noch im Reaktionsgemisch vorhandenes organisches Lösungsmittel abgetrennt werden.

Anschließend wird das Reaktionsgemisch mit einer derartigen Menge Wasser und einer derartigen Menge einer anorganischen Säure HX versetzt, dass mindestens 85 %, bevorzugt mindestens 90 % der Gesamtmenge des gebildeten sekundären Ammoniumsalzes des Bis-CTMA ausfällt, während mindestens 85 %, bevorzugt mindestens 90 % der Gesamtmenge des gebildeten primären Ammoniumsalzes des AMCT in Lösung verbleiben.

Als anorganische Säure HX kann z. B. HCl, H₂SO₄, H₃PO₄ oder HBr verwendet werden; bevorzugt wird HCl oder H₂SO₄ eingesetzt. Die anorganische Säure wird zweckmäßigerweise in Form wässriger Lösungen üblicher Konzentrationen eingesetzt, die entweder käuflich erhältlich oder aber durch Verdünnen leicht hergestellt werden können.

Die erforderliche Menge Wasser sowie die erforderliche Menge an Säure kann vom Fachmann leicht ermittelt werden. Bevorzugt wird dem Reaktionsgemisch eine gesamte Wassermenge (d.h. inklusive des über die eingesetzte anorganische Säure ins Reaktionsgemisch gelangenden Wassers) von 60 bis 200 ml, bevorzugt 100 bis 150 ml Wasser pro mol CCMT zugesetzt bzw. 55 bis 180 ml, bevorzugt 90 bis 135 ml Wasser, bezogen auf die gesamte Molmenge an gebildeten Aminothiazolen AMCT und Bis-CTMA.

Es kann ferner mit einem bis zu 30 mol-%igen Unterschuss oder Überschuss der anorganischen Säure, bezogen auf die gesamte Menge an gebildeten Aminothiazolen AMCT und Bis-CTMA gearbeitet werden. Bevorzugt wird mit 80 bis 100 mol-% anorganischer Säure, bezogen auf die gesamte Menge an gebildeten Aminothiazolen AMCT und Bis-CTMA gearbeitet. Hierbei stellt sich ein pH-Wert von 0 bis 3, bevorzugt von 1 bis 2 ein. Als Reaktionstemperatur hat sich eine Temperatur im Bereich von 10 bis 40 °C, bevorzugt von 25 bis 30 °C bewährt.

Beim Rühren des Reaktionsgemisches bilden sich die Salze der Amine AMCT und Bis-CTMA, beim bevorzugten Einsatz von HCl das Hydrochlorid. Hierbei fällt das gebildete sekundäre Ammoniumsalz zu mindestens 85 %, bevorzugt zu mindestens 90 % aus, während das primäre Ammoniumsalz zu mindestens 85 %, bevorzugt zu mindestens 90 %, und in hoher durch HPLC nachgewiesener Reinheit in der Mutterlauge verbleibt.

Die Löslichkeiten der Ammoniumsalze von AMCT und Bis-CTMA können innerhalb gewisser Grenzen durch die Einstellung des pH-Wertes, durch Salzzusätze (wie z. B. NaCl oder NH₄Cl) oder auch durch die Einstellung der Temperatur beeinflusst werden; dies ist dem durchschnittlichen Fachmann geläufig. Bewährt hat sich besonders der Zusatz von NaCl in einer Menge von 10 Gew.-% der gesamten Wasserphase.

Das ausgefallene Ammoniumsalz des Bis-CTMA kann durch Filtrieren, Zentrifugieren oder sonstige mechanische Trennverfahren praktisch vollständig vom Reaktionsgemisch abgetrennt werden. Die Verluste an AMCT bei dieser Abtrennung sind sehr gering und beeinträchtigen die Wirtschaftlichkeit des Verfahrens nicht.

Das bei der Abtrennung als Rückstand erhaltene Salz des Bis-CTMA kann beispielsweise mit Wasser, verdünnter Salzsäure, NaCl-Lösung oder NH₄Cl-Lösung nachgewaschen werden. Die dabei anfallenden Waschlösungen können mit dem vom Bis-CTMA befreiten und das AMCT enthaltenden Reaktionsgemisch vereinigt werden, um eine optimale AMCT-Ausbeute zu erhalten. Sollte eine noch weitergehendere Reinigung des Bis-CTMA gewünscht sein, ist dies beispielsweise durch Extraktion, Chromatografie, Kristallisation oder Umkristallisation möglich.

Man erhält eine saure, wässrige Lösung des Ammoniumsalzes des AMCT, die entweder direkt in der nächsten Reaktionsstufe weiterverwendet werden kann oder aber nach an sich bekannten Methoden (wie Eindampfen und folgendes Umkristallisieren oder Extraktion bzw. Reinigung durch Adsorption etc.) weiter gereinigt und isoliert werden kann. Eine solche Reinigung ist bevorzugt, wenn eine Ware von AMCT gewünscht wird, die weitgehend frei von anorganischen Salzen ist oder wenn nicht das Salz, sondern die freie Base AMCT erwünscht ist.

Dieses unterschiedliche Verhalten in der Löslichkeit der Ammonium-Salze von AMCT und Bis-CTMA ist bisher unbekannt, konnte nicht vorhergesehen werden und ermöglicht eine vorteilhafte Aufarbeitung der eingesetzten Reaktionsgemische. Über das erfindungsgemäße Verfahren wurde erstmals das Bis-CTMA gefunden und isoliert.

Das Bis-(2-chlor-thiazolyl-5-methyl)-amin sowie seine Salze, insbesondere das HCl-Salz sowie das Hydrat dieses HCl-Salzes, können als Zwischenprodukt zur Herstellung von Pflanzenschutzmitteln, von Pharmaka sowie von Textilfarbstoffen verwendet werden.

### Beispiel 1

Im Autoklaven legt man 19 g Wasser und 204 g (335 ml; 12,0 mol) Ammoniak vor und erwärmt auf 50°C. Innerhalb von ca. 1 Stunde pumpt man eine Lösung von 51,5 g CCMT (98 %ig; 0,30 mol) in 10 g Methyl-tert.-Butylether (MtBE) zu und spült die Pumpe mit ca. 52 g (70 ml) MtBE nach. Man rührt 1 Stunde nach; der Innendruck erreicht dabei maximal 20 bar. Man kühlt auf RT ab und entspannt langsam. Man nimmt den Autoklaveninhalt (86 g, zweiphasig) aus und destilliert im Wasserstrahlvakuum bei 22°C restliches NH₃ und MtBE ab. Man verdünnt zunächst mit 8 ml Wasser und säuert dann mit 20 ml (23,5 g; 0,24 mol) konzentrierter HCl bis auf einen pH Wert von 1 an. Man gibt noch 9 ml Wasser nach und erhält 106,8 g Lösung (B1 / 1). Beim Stehen fallen nach 1 bis 2 Stunden bei 25°C weiße Kristalle aus. Man filtriert ab, wäscht zweimal mit je 10 ml Eiswasser und erhält 11,6 g (feucht) bzw. 8,1 g B1 / tr. (=trocken) Feststoff sowie 84,3 g Lösung (B1 / ML) und 12,5 g Waschwasser 1 (B1 / W1) und 13,0 g Waschwasser 2 (B1 / W2).

Der Gehalt am AMCT bzw. Bis-CTMA in den unterschiedlichen Stufen wurde durch HPLC mit einer Genauigkeit von jeweils ca. 1 % bestimmt und ist in der nachfolgenden Tabelle 1 angegeben.

**Tabelle 1**

| Beispiel 1 | Auswaage [g] | Gehalt AMCT | | Gehalt Bis-CTMA | | Ausbeute | |
|---|---|---|---|---|---|---|---|
| | | [%] | [g] | [%] | [g] | AMCT [% d. Th.] | Bis-CTMA [% d. Th.] |
| B1 / 1 | 106,8 | 33,8 | 36,1 | 3,2 | 3,4 | 81,0 | 8,1 |
| B1 / tr. | 8,1 | 43,8 | 3,6 | 33,3 | 2,7 | 8,0 | 6,4 |
| B1 / ML | 84,3 | 34,5 | 29,1 | 0,2 | 0,2 | 65,2 | 0,4 |
| B1 / W1 | 12,5 | 20,0 | 2,5 | 1,5 | 0,2 | 5,6 | 0,4 |
| B1 / W2 | 13,0 | 12,6 | 1,6 | 2,2 | 0,3 | 3,7 | 0,7 |
| Produkt: Summe: ML + W1 + W2 | 109,8 | 29,6 | 32,5 | 0,55 | 0,55 | 72,9 | 1,3 |

### Beispiel 2

Die Durchführung erfolgt analog zu Beispiel 1, wobei jedoch mit verdoppeltem Einsatz gearbeitet wird und anstelle mit Wasser mit konzentrierter NaCl-Lösung nachgewaschen wird.

**Tabelle 2**

| Beispiel 2 | Auswaage [g] | Gehalt AMCT | | Gehalt Bis-CTMA | | Ausbeute | |
|---|---|---|---|---|---|---|---|
| | | [%] | [g] | [%] | [g] | AMCT [% d. Th.] | Bis-CTMA [% d. Th.] |
| B2 / ML | 169,2 | 36,2 | 61,3 | 0,2 | 0,3 | 68,7 | 0,3 |
| B2 / W1 | 19,8 | 24,3 | 4,8 | < 0,1 | < 0,01 | 5,4 | |
| B2 / W2 | 21,3 | 4,7 | 1,0 | <<0,1 | <0,01 | 1,1 | |
| Summe: ML + W1 + W2 | 210,3 | 31,5 | | 0,14 | | 74,3 | 0,2 |

Wie zu erkennen ist, liegt bei dieser Art der Nachwaschung mit NaCl-Lösung der Gehalt an Bis-CTMA in den Fraktionen, die das gewünschte AMCT enthalten, noch niedriger, es ist also eine noch bessere Abtrennung des Bis-CTMA und höhere Reinheit des AMCT möglich.

Man erhält AMCT als wässrige salzsaure Lösung, die laut HPLC eine 99 %ige Reinheit aufweist (Beurteilung als Summe der Flächen-% bei 226 nm Detektor-Wellenlänge).

### Beispiel 3

### Synthese von Bis-(2-chlor-thiazolyl-5-methyl)-amin und analytische Charakterisierung

17 g 2-Chlor-5-chlormethyl-thiazol (0,1 mol) werden in 3 g DMF gelöst und mit 135 g 26 %iger NH₃-Lösung (2 mol) versetzt. Man lässt 6 Tage bei RT stehen. Dabei bilden sich zwei Phasen. Man verdünnt mit 50 ml Wasser, säuert mit HCl (37 %) an (pH = 1) und erwärmt auf 55°C, wobei zwischendurch ausgefallene weiße Kristalle wieder in Lösung gehen. Nach Klärung mit Aktivkohle kristallisiert beim Abkühlen das Produkt in weißen Kristallen aus, die abfiltriert, mit Eiswasser gewaschen und bei 50°C im Vakuum getrocknet werden. Die Ausbeute beträgt 5,6 g (lt. CHN-Analyse 88,5 %ig freie Base bzw. 100 % HCl-Salz) und entspricht 0,0178 mol (35,6 % d. Th.). Bei längerem Stehen an der Luft zieht das Produkt Wasser an und bildet ein Mono-Hydrat.

Der Strukturbeweis erfolgt außerdem durch MS: m/e = 279/281/283, M+, 2 Cl; < 1 %; 244/246, 1 Cl, M-Cl, 8 %; 132/134, 1 Cl, 100 %. (Bei chemischer Ionisation wird der Peak bei 280/282/284 (M+1) als base-peak gefunden.)

1H-NMR (DMSO): = 3,43 (s, br., 2 H, H₂O); 4,44 (s, 4 H, -CH₂-N); 7,85 (s, 2 H, = CH-); 10,42(s, br., 2 H, NH₂).
(Dabei bedeutet br.: breites Signal; s bedeutet: Singulett).

In der chromatografischen Analyse (HPLC) ist die Retentionszeit identisch mit dem Produkt, das laut Beispiel 1 bzw. durch Filtration abgetrennt wurde.

## Patentansprüche

1. Verfahren zur Aufarbeitung von 5-Aminomethyl-2-chlor-thiazol und Bis-(2-chlor-thiazolyl-5-methyl)-amin enthaltenden Reaktionsgemischen, die durch Umsetzung von 5-Chlormethyl-2-chlorthiazol mit Ammoniak erhältlich sind, **dadurch gekennzeichnet, dass**
1) gegebenenfalls im Reaktionsgemisch vorhandener Ammoniak abgetrennt wird,
2) das Reaktionsgemisch mit einer derartigen Menge an Wasser und einer derartigen Menge einer anorganischen Säure HX versetzt wird, dass mindestens 85 % der Gesamtmenge des gebildeten Salzes des Bis-(2-chlor-thiazolyl-5-methyl)-amins ausfällt und mindestens 85 % der Gesamtmenge des gebildeten Salzes des 5-Aminomethyl-2-chlorthiazols in Lösung verbleiben, und
3) das ausgefällte Salz des Bis-(2-chlor-thiazolyl-5-methyl)-amins abgetrennt wird .

2. Verfahren nach Anspruch 1, **dadurch gekennzeichnet, dass** als anorganische Säure HX HCl, H₂SO₄, H₃PO₄ oder HBr verwendet wird.

3. Verfahren nach Anspruch 1 oder 2, **dadurch gekennzeichnet, dass** das Reaktionsgemisch mit einer derartigen Menge an Wasser und einer derartigen Menge einer anorganischen Säure HX versetzt wird, dass mindestens 90 % der Gesamtmenge des gebildeten Salzes des Bis-(2-chlor-thiazolyl-5-methyl)-amins ausfällt und mindestens 90 % der Gesamtmenge des gebildeten Salzes des 5-Aminomethyl-2-chlor-thiazols in Lösung verbleiben.

4. Verfahren nach einem oder mehreren der Ansprüche 1-3, **dadurch gekennzeichnet, dass** dem Reaktionsgemisch eine gesamte Wassermenge von 60 bis 200 ml, bevorzugt 100 bis 150 ml Wasser pro mol CCMT zugesetzt wird, beziehungsweise 55 bis 180 ml, bevorzugt 90 bis 135 ml Wasser, bezogen auf die gesamte Molmenge an gebildeten Aminothiazolen AMCT und Bis-CTMA.

5. Verfahren nach einem oder mehreren der Ansprüche 1 bis 4, **dadurch gekennzeichnet, dass** mit einem bis zu 30 gew.-%igen Unterschuss oder Überschuss der anorganischen Säure, bevorzugt mit 80 bis 100 Gew.% anorganischer Säure, bezogen auf die gesamte Menge an gebildeten Aminothiazolen AMCT und Bis-CTMA gearbeitet wird.

6. Verfahren nach einem oder mehreren der Ansprüche 1 bis 5, **dadurch gekennzeichnet, dass** sich ein pH-Wert von 0 bis 3, bevorzugt von 1 bis 2 einstellt.

7. Verfahren nach einem oder mehreren der Ansprüche 1 bis 6, **dadurch gekennzeichnet, dass** bei einer Reaktionstemperatur im Bereich von 10 bis 40°C, bevorzugt von 25 bis 30°C gearbeitet wird.

8. Verfahren nach einem oder mehreren der Ansprüche 1 bis 7, **dadurch gekennzeichnet, dass** in Schritt 2) dem Reaktionsgemisch Salze, bevorzugt NaCl oder NH₄Cl zugesetzt werden.

9. Verfahren nach einem oder mehreren der Ansprüche 1 bis 8, **dadurch gekennzeichnet, dass** das nach der Abtrennung in Schritt 3) erhaltene Salz des Bis-CTMA mit Wasser, verdünnter Salzsäure, NaCl-Lösung oder NH₄Cl-Lösung nachgewaschen wird.

10. Bis-(2-chlor-thiazolyl-5-methyl)amin der Formel 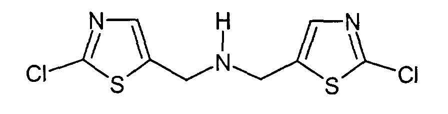 und seine Salze, bevorzugt das Chlorid und Sulfat.

11. Verwendung von Bis-(2-chlor-thiazolyl-5-methyl)-amin oder seinen Salzen, bevorzugt des HCl-Salzes und des Hydrats dieses HCI-Salzes, als Zwischenprodukt zur Herstellung von Pflanzenschutzmitteln, von Pharmaka oder von Textilfarbstoffen.
